# EUROPÄISCHE PATENTANMELDUNG

(11) **EP 1 795 584 A1**
(43) Veröffentlichungstag der Anmeldung: **13.06.2007**
(21) Anmeldenummer: 05027050.3
(22) Anmeldetag: 10.12.2005
(51) Int. Cl.: C12M 1/113, C12M 1/107, C12M 1/16

(54) **Einstufiges Betriebsverfahren für eine Durchfluss-Nassfermentations-Biogasanlage**

(71) Anmelder: U.T.S. Umwelt-Technik-Süd GmbH, 84419 Obertaufkirchen (DE)
(72) Erfinder: Bürger, Adam, 83527 Haag/Obb. (DE)
(74) Vertreter: Neubauer, Hans-Jürgen

(57) **Zusammenfassung**

Die Erfindung betrifft ein Betriebsverfahren zur Nassfermentation in einer einstufigen, eingefahrenen und im Durchflussverfahren arbeitenden Biogasanlage (1), mit wenigstens einem ein Substrat mit bestimmtem Trockensubstanzgehalt enthaltenen Fermenter (3) mit einer vorgeschalteten Zudosiereinrichtung (2) für vergärbare Stoffe und mit einem nachgeordneten Separator (8), mit dem eine zumindest teilweise Trennung eines aus dem Fermenter (3) abgezogenen Substrats in einen Substrat-Feststoffanteil (9) und einen Substrat-Flüssigkeitsanteil (11) erfolgt. Erfindungsgemäß wird mit der Zudosiereinrichtung (2) eine vorgegebene Menge an vergärbaren Stoffen in den Fermenter (3) eingebracht und eine entsprechende Menge an Substrat dem Fermenter (3) durch Verdrängung oder Pumpen entnommen und zumindest teilweise dem Separator (8) zugeführt. Weiter ist eine Trockensubstanzregelung für das im Fermenter (3) befindliche Substrat vorgesehen. Dazu wird ein bestimmter Soll-Trockensubstanzgehalt im Substrat des Fermenters (3) vorgegeben, der Ist-Trockensubstanzgehalt im Substrat des Fermenters (3) erfasst, und bei einer Überschreitung des Soll-Trockensubstanzgehalts durch den erfassten Ist-Trockensubstanzgehalt dieser auf den Soll-Trockensubstanzgehalt dadurch zurückgeführt, dass die erforderliche Regel-Flüssigkeitsmenge zur Verdünnung in den Fermenter (3) aus dem am Separator (8) anfallenden Substrat-Flüssigkeitsanteil (11) direkt oder indirekt zurückgeführt und eingespeist wird, so dass keine Zugabe zusätzlicher Fremdflüssigkeit erfolgt.

## Beschreibung

Die Erfindung betrifft ein Betriebsverfahren zur Nassfermentation in einer einstufigen, eingefahrenen und im Durchflussverfahren arbeitenden Biogasanlage nach dem Oberbegriff des Anspruchs 1.

Die Biogasgewinnung zur Energieerzeugung gewinnt vorrangig im landwirtschaftlichen Bereich zunehmend an Bedeutung. Die Gründe dafür sind im wesentlichen die Erschließung neuer Einkommensquellen in der Landwirtschaft durch eine staatlich geförderte regenerative und umweltverträgliche Energiegewinnung. Die dafür bekannten Biogasanlagen sind meist für einen kontinuierlichen einstufigen Betrieb ausgelegt, mit einem Fermenterbehälter, in dem in einem anaeroben Nassvergärungsprozess zur Gewinnung von Biogas Biomassen als anorganische Substrate vergoren werden. Je nach den Gegebenheiten sind einem solchen Fermenter ein Nachfermenter und ein Endlager sowie separate Gasspeicher für das erzeugte Biogas nachgeordnet. Zur Beschickung des Fermenterbehälters mit vergärbaren Stoffen ist diesem eine meist automatisierte Zudosiereinrichtung vorgeschaltet, wobei für flüssige Substratbestandteile Pumpen und für feste Biomassen Feststoff-Zudosiereinrichtungen mit Wägeeinrichtungen und Befüllschnecken oder Förderbändern verwendet werden. Zudem sind in den Behältern und Fermentern Rühreinrichtungen für eine gleichmäßige Verteilung der Substratbestandteile angebracht (EP 1 251 165 A1).

Die Erzeugung von Biogas erfolgt bei der anaeroben Nassvergärung im wesentlichen in vier Schritten, der Hydrolyse, der Versäuerungsphase (Acidogenese), der Essigsäurebildung (Acetogenese) und der Methanogenese.

Bei der dem erfindungsgemäßen Betriebsverfahren zugrundeliegenden einstufigen Biogasanlage laufen die vorstehenden Prozesse ohne räumliche Trennung gemeinsam und parallel ab. Ein solcher stabiler, eingefahrener und kontinuierlicher Prozessverlauf wird jedoch erst nach einer Einfahrphase erreicht, die nicht Gegenstand des vorliegenden Betriebsverfahrens ist.

Ein wesentlicher Aspekt der Biogasgewinnung ist die Nutzung zur Einspeisung in ein der Biogasanlage zugeordnetes Blockheizkraftwerk für eine wirtschaftlich lukrative Erzeugung von Elektroenergie, welche über staatlich festgelegte Vergütungssätze gefördert und unterstützt wird.

Der vorstehende, einstufige und kontinuierliche Betrieb zur Nassfermentation ist bei Verwendung des Durchflussverfahrens mit einem relativ einfachen und kostengünstigen Anlagenaufbau zu realisieren und durch Automatisierungsmöglichkeiten mit angemessenem Aufwand durchzuführen.

Dabei ist es für einen störungsfreien Gärprozess und Anlagenbetrieb wesentlich im Durchflussverfahren ein möglichst gleiches Füllniveau im Fermenter und ggf. in einem nachgeordneten Nachfermenter aufrecht zu erhalten. Dazu wird die durch eine kontinuierliche oder chargenweise, quasi kontinuierliche Beschickung mittels der Zudosiereinrichtung dem Fermenter zugeführte Beschickungsmenge als entsprechende, zumindest teilweise vergorene Substratmenge dem Fermenter entnommen. Diese Entnahme erfolgt in bekannter Weise durch einen Überlauf oder durch einen Pumpvorgang in einen Nachfermenter oder ein Endlager.

Bei der hier verfahrensmäßig zugrundeliegenden Nassfermentation enthält der Fermenter einen Trockensubstanzgehalt von maximal etwa 15%, bei dem das Substrat für die vorstehende einfache Entnahme zum Füllniveauausgleich fließ- und pumpfähig ist. Durch diese Entnahmeart wird dem Fermenter relativ viel Substrat-Flüssiganteil entnommen. Insbesondere bei einer Beschickung mit hohem Feststoffanteil oder bei einer ausschließlichen Feststoffbeschickung würde innerhalb kurzer Zeit der Trockensubstanzgehalt unzulässig hoch ansteigen. Um dies zu unterbinden, wird regelmäßig Flüssigkeit zur Verdünnung bei der Beschickung mit zugeführt.

Bei landwirtschaftlichen Betrieben mit Tierhaltung ist dies kein Problem, da anfallende Gülle als Verdünnungsflüssigkeit zusammen mit festen Biomassen ggf. nach einem Anmaischen in einer Anmaischgrube zugeführt werden können.

Viele landwirtschaftliche Betriebe haben jedoch keine Tierhaltung, so dass dort keine Gülle zur Verfügung steht. Bei dort installierten Biogasanlagen wird zur Verdünnung üblicherweise Wasser, meist Wasser aus der Trinkwasserversorgung beigemischt. Dies ist einerseits unwirtschaftlich und zudem wenig umweltverträglich.

Es ist zudem bekannt, einem Fermenter einen Separator nachzuordnen, mit dem das für einen Niveauausgleich abgezogene Substrat zumindest teilweise in einen Substrat-Feststoffanteil und einen Substrat-Flüssiganteil separiert wird. Ziel der bekannten Fest-Flüssig-Trennung durch einen nachgeschalteten Separator ist es aus dem abgezogenen Substrat einen hohen Substrat-Feststoffanteil auszuscheiden und diesen nicht mehr einem Endlager zuzuführen. Damit wird eine einfachere Endlagerung des Substrat-Flüssiganteils ohne ungünstige Schwimmschichten und Sedimentschichten aus entmischten Feststoffen möglich, wobei der separierte Feststoffanteil mit wenig Volumen gelagert, kompostiert oder als Dünger ausgebracht werden kann. Zudem ist es bekannt, das bei der Fest-Flüssig-Trennung am Separator anfallende Efluent unspezifisch zum Teil wieder als Bestandteil einer Anmaischflüssigkeit in Verbindung mit Gülle und/oder Wasser zur Verdünnung dem Fermenter zuzuführen. Trotz dieser bekannten Möglichkeit einer Teilrückführung von Efluent werden die vorstehenden Nachteile in Verbindung mit der Zugabe von Fremdflüssigkeit nicht behoben, sondern allenfalls reduziert, insbesondere wenn keine Gülle für eine Verdünnung zur Verfügung steht und Trinkwasser in Verbindung mit rückgeführtem Efluent zur Verdünnung verwendet wird.

Weiter ist ein grundsätzlich anderes Verfahren zur Biogasgewinnung aus Biomasse mit einer diskontinuierlichen Beschickung als sogenanntes Batchverfahren bekannt. Dabei wird ein Fermenter komplett mit frischem Substrat gefüllt und luftdicht verschlossen, wobei das Substrat bis zum Ende der gewählten Verweildauer im Behälter bleibt, ohne dass eine weitere Beschickung mit vergärbaren Stoffen oder eine Substratentnahme erfolgt. Dabei erfolgt keine Zugabe von Fremdflüssigkeit, insbesondere keine Zugabe von Trinkwasser, was von staatlicher Seite als gesteigert umweltverträglich gewertet und mit einem sogenannten Technologiebonus honoriert wird. Nachteilig ist beim Batchverfahren aber keine konstante Biogasproduktion und Biogasqualität gegeben.

Aufgabe der Erfindung ist es daher, ein Betriebsverfahren vorzuschlagen, mit dem der wirtschaftliche Betrieb und die Umweltverträglichkeit einer mit Nassfermentation im einstufigen Durchflussverfahren arbeitenden, eingefahrenen Biogasanlage verbessert werden.

Diese Aufgabe wird mit den Merkmalen des Anspruchs 1 gelöst.

Gemäß Anspruch 1 wird mit der Zudosiereinrichtung eine vorgegebene Menge an vergärbaren Stoffen kontinuierlich oder chargenweise quasi kontinuierlich in den Fermenter eingebracht und zur Aufrechterhaltung eines im wesentlichen gleichen Füllniveaus im Behälter eine entsprechende Menge an Substrat aus dem Fermenter durch Verdrängung oder Pumpen entnommen, wobei dieses Substrat zumindest teilweise dem Separator zugeführt wird. Der hier betrachtete eingefahrene Betriebszustand der Biogasanlage umfasst auch den Betrieb einer im Fermenter installierten Rührtechnik, so dass die Angaben und Betrachtungen zum Trockensubstanzgehalt hier insbesondere ein betriebsmäßig gerührtes und durchmischtes Substrat betreffen.

Weiter ist eine Trockensubstanzregelung für das Substrat im Fermenter vorgesehen. Dazu wird ein bestimmter Soll-Trockensubstanzgehalt im Substrat des Fermenters vorgegeben, bei dem insbesondere das Substrat für eine Entnahme zum Niveauausgleich auch bei ggf. hohem Trockensubstanzgehalt noch fließ- und pumpfähig ist. Zudem wird der Ist-Trockensubstanzgehalt im Substrat des Fermenters erfasst und bei einer Überschreitung des Trockensubstanzgehalts durch den erfassten Ist-Trockensubstanzgehalt wird dieser auf den vorgegebenen Trockensubstanzgehalt durch Flüssigkeitszugabe zurückgeführt, indem die erforderliche Regel-Flüssigkeitsmenge zur Verdünnung in den Fermenter aus dem am Separator anfallenden Substrat-Flüssigkeitsanteil direkt oder indirekt zurückgeführt und eingespeist wird.

Damit wird auch eine einstufige, zur Nassfermentation im Durchflussverfahren arbeitende Biogasanlage im eingefahrenen Betriebszustand ohne Zugabe von Fremdflüssigkeit betreibbar. Ohne Zugabe von Fremdflüssigkeit kann eine solche Biogasanlage vorteilhaft in Verbindung mit landwirtschaftlichen Betrieben, die keine Viehhaltung betreiben und damit keine Gülle zur Verfügung haben, eingesetzt werden. Insbesondere ist kein Frischwasser als Fremdflüssigkeit erforderlich, was zur Senkung der Betriebskosten und zur Erhöhung der Umweltfreundlichkeit beiträgt. Zudem kann bei diesem Betrieb der staatliche Technologiebonus für eine weitere Steigerung der Wirtschaftlichkeit in Anspruch genommen werden, der als Aufschlag pro elektrisch erzeugter Energieeinheit ausgezahlt wird.

Eine Automatisierung der Trockensubstanzregelung mit einer Trockensubstanz-Regeleinrichtung reduziert den für die Biogasanlage erforderlichen Arbeitsaufwand. Die Trockensubstanz-Regelung kann dabei ggf. in Verbindung mit weiteren erforderlichen Regelungen in entsprechenden Geräten und Schrankanordnungen integriert werden. Durch die Möglichkeit der Vorgabe eines Soll-Trockensubstanzgehalts kann dieser auf die jeweiligen Gegebenheiten optimiert werden, insbesondere zur Optimierung der Vergärung, für ein weitgehend gleiches Fermenterfüllniveau und zur Gewährleistung der Fließfähigkeit und Pumpfähigkeit des Substrats im Fermenter.

Zudem ergeben sich Vorteile durch die erforderliche Fest-Flüssigkeits-Trennung mittels des Separators, wobei insbesondere der prozentuale Feststoffanteil in einem nachgeordneten Endlager reduzierbar ist. Dadurch kann dort ggf. die Installation einer Rührtechnik und deren Betriebsdauer kostengünstig reduziert werden und durch hohe Feststoffgehalte bedingte Probleme bei der Entleerung des Endlagers, treten nicht mehr auf. Der nach dem Separator anfallende Substrat-Feststoffanteil kann einfach durch Lagerung oder Ausbringung verwertet werden.

Das vorstehende Betriebsverfahren ist mit den angegebenen Vorteilen entsprechend bei parallel beschickbaren Fermentern und/oder Nachfermentern anwendbar.

Gemäß Anspruch 2 sollen mit der Zudosiereinrichtung in der Art eines an sich bekannten Feststoffzudosierers nur Feststoffe zudosiert werden, insbesondere gemäß Anspruch 3 ausschließlich nachwachsende Rohstoffe (NaWaRo). Die Erfindung macht sich dabei die Kenntnis zu Nutze, dass in Feststoff-Biomassen insbesondere bei einer Beschickung der Biogasanlage mit nachwachsenden Rohstoffen gebundene Flüssigkeiten beispielsweise als Zellwasser enthalten sind und dem Fermenter zugeführt werden. Diese gebundenen Flüssigkeiten werden durch den Gärprozess weitgehend freigesetzt, so dass pro zudosierter Feststoffmengeneinheit jeweils auch ein bestimmter, darin enthaltener Flüssigkeitsanteil im Fermenter freigesetzt wird und für eine Verwertung als Substrat-Flüssigkeitsanteil zur Verfügung steht. Insbesondere bei NaWaRos wird im Gärprozess mehr gebundener Flüssiganteil freigesetzt, als in einer Langzeitbetrachtung für die erfindungsgemäße Rückführung als Regel-Flüssigkeitsmenge benötigt wird, so dass in jedem Fall eine ausreichende Regel-Flüssigkeitsmenge nach dem Separator zur Verfügung steht und der Rest einem Endlager zugeführt oder als Flüssigdünger ausgebracht werden kann.

Die ausschließliche Beschickung mit NaWaRo's hat zudem den Umweltschutzvorteil, dass mit der daraus erfolgten Energiegewinnung keine Erhöhung des CO₂-Gehalts bei einer Gesamtbetrachtung des CO₂-Kreislaufs verbunden ist. Dieser Umweltschutzvorteil wird staatlich durch Zuwendungen honoriert, wobei durch die erfindungsgemäße Vermeidung einer Fremdflüssigkeits-Zudosierung noch der sogenannte Technologiebonus dazukommt.

Mit den Merkmalen des Anspruchs 4 wird eine im Betriebsverfahren konkret einsetzbare, vollautomatisierbare, elektrisch arbeitende Trockensubstanz-Regeleinrichtung beansprucht. Zur Ermittlung des Ist-Trockensubstanzgehalts wird eine Sonde verwendet, die nach üblichen Messmethoden kapazitiv, induktiv, optisch, durch Ultraschalltechnik oder mit ähnlichen Verfahren arbeiten kann. Vom Regler wird nach Bewertung einer Regelabweichung über einen Regelalgorithmus ein Stellsignal an ein Regelstellglied zur Bereitstellung und Zuführung der Regelflüssigkeitsmenge abgegeben. Das Regelstellglied kann hier beispielsweise als Dosierpumpe ausgebildet sein oder als steuerbares Durchflussventil, welches aus einem Flüssigkeitsstrom des Substrat-Flüssigkeitsanteils die erforderliche Regel-Flüssigkeitsmenge zur Zuführung in den Fermenter freigibt.

Eine effektive Nassfermentation in einem Durchflussverfahren ist nach derzeitigem allgemeinen Kenntnisstand bei einem Trockensubstanzgehalt von nicht wesentlich mehr als 15% im Substrat möglich. Entsprechend soll nach Anspruch 5 der Soll-Trockensubstanzgehalt mit kleiner oder gleich 15,5% vorgegeben werden. Da die Gasausbeute bei einem hohen Trockensubstanzgehalt steigt, wird vorgeschlagen diesen als Soll-Trockensubstanzgehalt zwischen 10% und 15,5% vorzugeben.

Gemäß Anspruch 6 kann das dem Separator zugeführte Substrat unmittelbar dem Fermenter und/oder einem dem Fermenter nachgeordneten Nachfermenter entnommen werden, wobei bei einer Zudosierung vorerst über die Anordnung Fermenter-Nachfermenter ein Niveauausgleich erfolgt und ggf. etwas zeitversetzt Substrat aus dem Nachfermenter abgezogen wird.

In einer Verfahrensführung nach Anspruch 7 wird dem Separator die gesamte aus dem Fermenter oder ggf. dem Nachfermenter entnommene Substratmenge zugeführt. Aus dem nach dem Separator anfallenden Substrat-Flüssigkeitsanteil wird dann eine Teilmenge als Regel-Flüssigkeitsmenge abgezweigt und in den Fermenter zurückgeführt. Die verbleibende Flüssigkeits-Restmenge wird einem Endlager zugeführt oder auf Freiflächen ausgebracht. Alternativ dazu kann nach Anspruch 8 auch die erforderliche Regel-Flüssigkeitsmenge dadurch gewonnen und zur Verfügung gestellt werden, dass nur die dazu erforderliche Substratmenge für eine Fest-Flüssig-Trennung dem Separator zugeführt wird.

Je nach den Gegebenheiten und den für die Beschickung verwendeten Biostoffen kann es nach Anspruch 9 zweckmäßig sein, die Regel-Flüssigkeitsmenge unmittelbar in den Fermenter zurückzuführen, vorzugsweise zurückzupumpen. Alternativ dazu kann eine Rückführung auch über eine Vorgrube als Vorratsgefäß und/oder eine Anmaischgrube erfolgen. Dabei ist die Regelung und Flüssigkeitsbilanzierung mit der Flüssigkeitsverdünnung ohne Fremdflüssigkeit durch die Zwischenschaltung von Vorratsmengen über einen etwas längeren Zeitraum zu betrachten.

Als Separatoren können nach Anspruch 10 je nach den Anlagebedingungen und Betriebsbedingungen an sich bekannte Schrauben- bzw. Schnecken-Separatoren und/oder Zentrifugen-Separatoren und/oder Siebbandpressen verwendet werden, wobei jeder Separatortyp hinsichtlich der Effektivität der Separierung und des Betriebs und der Wartung unterschiedliche an sich bekannte Eigenschaften aufweist, die bei einer Entscheidung für einen bestimmten Separatortyp zu berücksichtigen sind.

Als alternative oder zusätzliche Separatoranordnung wird nach Anspruch 11 eine Schüttung aus Biofeststoffen, beispielsweise aus Stroh als Feststofffilter vorgeschlagen, über die das abgezogene Substrat geleitet wird. Die Schüttung hält dabei einen Substrat-Feststoffanteil zurück und nimmt diesen auf, wobei ein Substrat-Flüssigkeitsanteil durch die Schüttung durchsickert und für die weitere Verarbeitung, insbesondere als Regel-Flüssigkeitsmenge aufgefangen und zur Verfügung gestellt werden kann. Die Schüttung kann zusammen mit den darin separierten Substrat-Feststoffanteilen dem Fermenter als Beschickungsmaterial zugeführt werden.

Der hier vorliegende Trockensubstanzausgleich ist regelungstechnisch gesehen relativ träge. Um aber dennoch einen relativ schnellen Ausgleich einer Trockensubstanzabweichung zu erreichen, wird mit Anspruch 12 vorgeschlagen in einem Regelalgorithmus einen Differenzialanteil vorzusehen und/oder eine Regelung mit einer Steuerung für eine schnelle Grobanpassung zu kombinieren. Beispielsweise könnte dazu für eine Grobanpassung bei einer Zudosierung einer bestimmten Feststoffmenge sofort eine angepasste, separierte Substrat-Flüssigkeitsmenge zurückgeführt werden, die jedoch noch eine Verdünnung unterhalb des vorgegebenen Sollwerts bewirkt, wobei dann eine Ausregelung der Regelabweichung über eine Regeleinrichtung erfolgen kann.

In einer besonders bevorzugten Ausgestaltung des Betriebsverfahrens nach Anspruch 13 wird zumindest ein Teil des durch den Separator separierten Substrat-Feststoffanteils über die Zudosiereinrichtung in den Fermenter zurückgeführt. Dem liegt die Erkenntnis zugrunde, dass bei einem Durchflussverfahren im aus dem Fermenter abgezogenen Substrat zwangsläufig noch ein hoher Anteil weitervergärbarer Feststoffanteile enthalten ist. Dies ist auch dann der Fall, wenn Substrat erst aus dem Nachfermenter abgezogen wird. Zudem sind in den separarierten Substrat-Feststoffanteilen auch Bakterien enthalten, die bei einer Rückführung die Weitervergärung begünstigen. Das abgezogene Substrat kann nicht insgesamt in den Fermenter zurückgeführt werden, da dann das Füllniveau wegen der zusätzlichen Beschickungsmenge kontinuierlich steigen würde. Anders liegt der Fall jedoch nach einer Fest-FlüssigkeitsTrennung nach dem Separator, da im wesentlichen kleineren Volumen des separierten Substrat-Feststoffanteils ein hoher Anteil von noch weitervergärbarer Stoffe enthalten ist. Daher wird mit Anspruch 14 vorgeschlagen, den gesamten separierten Substrat-Feststoffanteil wieder vorzugsweise automatisiert über die Zudosiereinrichtung dem Fermenter zuzuführen. Der separierte Substrat-Feststoffanteil wird zusammen mit einer relativ geringen Anteilsmenge als Regel-Flüssigkeitsmenge zur Verdünnung dem Fermenter zurückgeführt, während die regelmäßig dagegen größere Restmenge an separierter Substratflüssigkeit als Efluent vorzugsweise automatisiert einem Endlager zugeführt oder ausgebracht wird. Bei dieser Verfahrensführung wird die Energieausbeute aus den für die Vergärung eingesetzten Biostoffen insbesondere von NaWaRos bei geeigneten Verweildauern im Fermenter erhöht und damit insgesamt die Wirtschaftlichkeit der Biogasanlage verbessert.

Anhand einer Zeichnung wird die Erfindung näher erläutert.

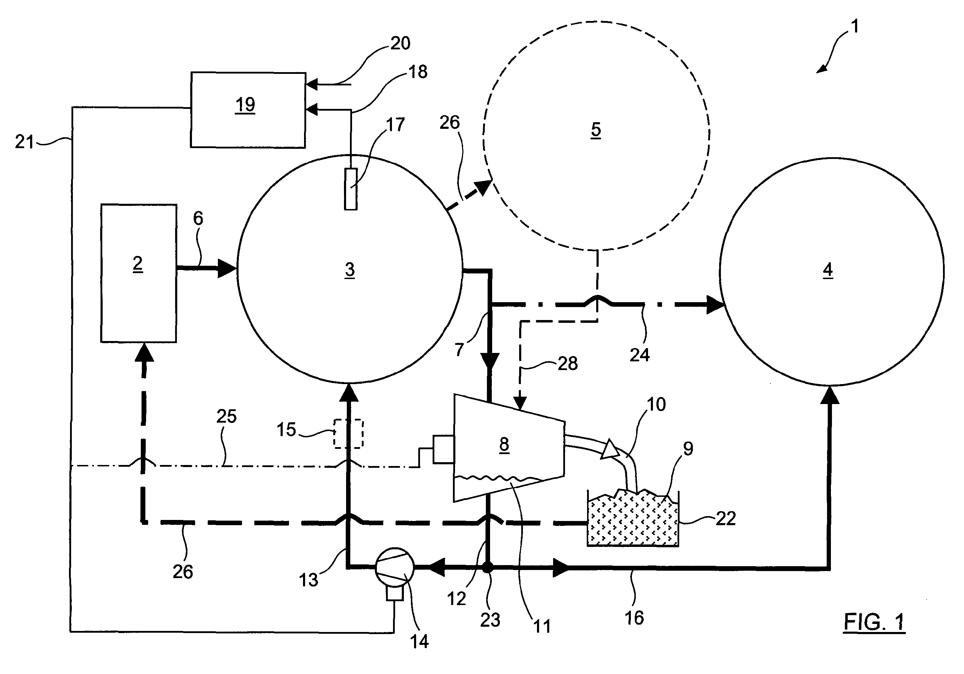

In der einzigen Figur ist schematisch eine einstufig betrieben im Durchflussverfahren arbeitende Biogasanlage 1 für eine Nassfermentation schematisch in einer Draufsicht gezeigt.

Die Biogasanlage 1 umfasst eine Feststoff-Zudosiereinrichtung 2, einen nachgeordneten Fermenter 3, dem ein Endlager 4 für Gärsubstratreste nachgeordnet ist. Dazwischen kann je nach Ausführung der Biogasanlage 1 noch ein (strichliert dargestellter) Nachfermenter 5 eingeschaltet sein.

Die Feststoff-Zudosiereinrichtung 2 ist für die Beschickung des Fermenters 3 mit nachwachsenden Rohstoffen (NaWaRos) sowie für eine kontinuierliche oder chargenweise, quasi kontinuierliche und mengenmäßig mit Hilfe von Wägeeinrichtungen vorbestimmte Beschickung ausgelegt. Solche Feststoff-Zudosiereinrichtungen für Biogasanlagen sind in unterschiedlichen Ausführungen aus dem Stand der Technik bekannt.

Fermenter, Nachfermenter und Endlager können in unterschiedlichen an sich bekannten Ausführungsformen verwendet werden, insbesondere als Betonbehälter oder Stahlbehälter in einer Unterflur- oder Oberfluranordnung.

Die Beschickung des Fermenters 3 mit NaWaRos ist schematisch durch den Pfeil 6 angegeben. Um im eingefahrenen Zustand der Biogasanlage 1 den Füllstand des Fermenters 3 und ggf. des Nachfermenters 5 auf gleichem Niveau zu halten, ist bei einer Beschickung mit NaWaRos Substrat durch Pumpen oder durch einen Überlauf in der entsprechenden Menge aus dem Fermenter 3 über die schematisch angegebene Leitung 7 oder ggf. aus dem Nachfermenter 5 über die (strichliert eingezeichnete) Leitung 28 abzuziehen. Das Substrat ist dabei noch nicht vollständig vergärt.

Dieses abgezogene Substrat wird einem Separator 8 zugeführt, der eine Fest-Flüssigkeits-Trennung in einen Substrat-Feststoffanteil 9, dargestellt über den Auswurf 10, und einen Substrat-Flüssiganteil, der aufgefangen und durch die Leitung 12 strömt, durchführt. Die Leitung 12 verzweigt sich an einer Verzweigungsstelle 23 in eine Leitung 13, die über eine ansteuerbare Dosierpumpe 14 und ggf. über eine (strichliert eingezeichnete) Vorgrube 15 mit dem Fermenter 3 verbunden ist und in eine Leitung 16, die Verbindung mit dem Endlager 4 hat.

Im Fermenter 3 ist im dargestellten Ausführungsbeispiel eine schematisch dargestellte Sonde 17 zur Erfassung eines Ist-Trockensubstanzgehalts eingebracht, deren Messwert als Istwert (Pfeil 18) einem Regler 19 zugeführt ist, an dem zudem ein Soll-Trockensubstanzgehalt (Pfeil 20) vorgebbar ist. Bei einer Regelabweichung wird vom Regler 19 über die Leitung 21 ein bewertetes Stellsignal für eine ermittelte Mengenzudosierung eines Substrat-Flüssiganteils 11 an die Dosierpumpe 14 abgegeben.

Die in der Figur mit durchgehend gezeichneten Leitungslinien dargestellte Ausführungsform hat folgende Funktion:

Entsprechend der mit der Feststoff-Zudosiereinrichtung 2 zudosierten Biomassenmenge wird über die Leitung 7 Substrat aus dem Fermenter 3 abgezogen und dem Separator 8 zugeführt, wo die gesamte abgezogene Substratmenge separiert wird. Der separierte Substrat-Feststoffanteil 9 wird in der dargestellten Ausführung in einem Behälter 22 für eine weitere Verwertung aufgefangen. Der gesamte separierte Substrat-Flüssigkeitsanteil 11 wird an der Verzweigungsstelle 23 aufgeteilt in die von der Regelung für eine Verdünnung und Ausregelung auf den vorgegebenen Soll-Trockensubstanzgehalt erforderliche Regel-Flüssigkeitsmenge, die mittels der Dosierpumpe 14 über die Leitung 13 ggf. über die Vorgrube 15 dem Fermenter 3 zugeführt wird. Über die Leitung 16 wird (ggf. mit einer nicht eingezeichneten Pumpe) die verbleibende Restmenge in das Endlager 4 als Efluent gepumpt. Da der Separator 8, unabhängig davon, welcher von den an sich bekannten Separtortypen eingesetzt wird, keine vollständige Fest-Flüssig-Trennung durchführen kann, wird je nach Separationsgrad mit dem Efluent auch ein bestimmter Feststoffanteil dem Endlager 4 zugeführt.

Die vorbeschrieben Ausführung kann so modifiziert werden, dass nicht das gesamte aus dem Fermenter abgezogene Substrat dem Separator 8 zugeführt wird, sondern über die strichpunktierte Leitung 24 ein Teil unmittelbar in das Endlager 4 geleitet wird. Dabei muss jedoch sichergestellt werden, dass die zur Regelung des eingestellten Trocken-Substanzgehalts angeforderte Regel-Flüssigkeitsmenge nach dem Separator 8 in jedem Fall zur Verfügung steht. Die Laufzeit des Separators 8 kann zur Einsparung von Energie zeitlich in Abstimmung mit der Zudosierung bzw. dem Abzug von Substrat und/oder dem Bedarf an Regel-Flüssigkeitsmenge für die Verdünnung gesteuert werden, was schematisch durch die Steuerleitung 25 angegeben ist.

In der in der Figur strichliert eingezeichneten Weiterbildung mit dem Nachfermenter 5 wird bei einer Beschickung durch die Feststoff-Zudosiereinrichtung 2 eine entsprechende Menge Substrat über die strichliert eingezeichnete Leitung 26 als Pumpleitung oder Überlaufleitung dem Nachfermenter 5 zugeführt und von dort die entsprechende Substratmenge abgezogen und über die Leitung 28 dem Separator 8 zugeführt, wobei die Leitung 7 entfällt. Die weitere Verarbeitung des abgezogenen Substrats verläuft wie vorstehend beschrieben.

In einer besonders bevorzugten Weiterbildung wird der separierte Substrat-Feststoffanteil 9 insgesamt wieder dem Fermenter 3 über die Feststoff-Zudosiereinrichtung 2 zugeführt, was durch den strichliert eingezeichneten Förderweg 26 schematisch dargestellt ist. Zweckmäßig erfolgt diese Rückführung des gesamten Substrat-Feststoffanteils 9 zur Feststoff-Zudosiereinrichtung 2 durch bekannte Fördermittel, wie Förderbänder etc. Die Rückführung kann jedoch auch chargenweise durch Transportmittel mit Ladeeinrichtung, wie z. B. Frontlader erfolgen.

Der wesentliche Aspekt bei der im Durchflussverfahren betriebenen Biogasanlage 1 ist, dass keine Fremdflüssigkeit im eingefahrenen Betrieb zudosiert wird, sondern die für die erforderliche Substratverdünnung im Fermenter benötigte Regel-Flüssigkeitsmenge aus dem Prozess nach dem Separator 8 gewonnen und genau in der erforderlichen Menge zugegeben wird.

## Patentansprüche

1. Betriebsverfahren zur Nassfermentation in einer einstufigen, eingefahrenen und im Durchflussverfahren arbeitenden Biogasanlage (1),
mit wenigstens einem ein Substrat mit bestimmtem Trockensubstanzgehalt enthaltenden Fermenter (3) mit einer vorgeschalteten Zudosiereinrichtung (2) für vergärbare Stoffe und mit einem nachgeordneten Separator (8), mit dem eine zumindest teilweise Trennung eines aus dem Fermenter (3) abgezogenen Substrats in einen Substrat-Feststoffanteil (9) und einen Substrat-Flüssigkeitsanteil (11) erfolgt,
**dadurch gekennzeichnet,**
**dass** mit der Zudosiereinrichtung (2) eine vorgegebene Menge an vergärbaren Stoffen kontinuierlich oder chargenweise quasi kontinuierlich in den Fermenter (3) eingebracht wird und eine entsprechende Menge an Substrat dem Fermenter (3) durch Verdrängung oder Pumpen entnommen und zumindest teilweise dem Separator (8) zugeführt wird,
**dass** eine Trockensubstanzregelung für das im Fermenter (3) befindliche Substrat vorgesehen ist dergestalt,
**dass** ein bestimmter Soll-Trockensubstanzgehalt im Substrat des Fermenters (3) vorgegeben wird, bei dem insbesondere das Substrat auch bei ggf. hohem Trockensubstanzgehalt noch fließ- und pumpfähig ist,
**dass** der Ist-Trockensubstanzgehalt im Substrat des Fermenters (3) erfasst wird,
**dass** bei einer Überschreitung des Soll-Trockensubstanzgehalts durch den erfassten Ist-Trockensubstratgehalt dieser auf den Soll-Trockensubstanzgehalt zurückgeführt wird, indem die erforderliche Regel-Flüssigkeitsmenge zur Verdünnung in den Fermenter (3) aus dem am Separator (8) anfallenden Substrat-Flüssigkeitsanteil (11) direkt oder indirekt zurückgeführt und eingespeist wird, so dass keine Zugabe zusätzlicher Fremdflüssigkeit erfolgt.

2. Betriebsverfahren nach Anspruch 1, **dadurch gekennzeichnet, dass** mit der Zudosiereinrichtung als Feststoffzudosierer nur Feststoffe zudosiert werden, wobei durch den Gärprozess darin gebundene Flüssigkeitsanteile freigesetzt werden.

3. Betriebsverfahren nach Anspruch 1 oder 2, **dadurch gekennzeichnet, dass** als vergärbare Stoffe ausschließlich nachwachsende Rohstoffe (NaWaRos) zudosiert werden.

4. Betriebsverfahren nach einem der Ansprüche 1 bis 3, **dadurch gekennzeichnet, dass** in einer Trockensubstanz-Regeleinrichtung der Ist-Trokkensubstanzgehalt im Substrat des Fermenters (3) über eine Sonde (17) gemessen wird und der Messwert als elektrisches Ist-Signal einem Regler (19) zugeführt wird, an dem zudem der Soll-Trockensubstanzgehalt über ein elektrisches Soll-Signal (Pfeil 20) einstellbar ist, und
dass vom Regler (19) nach Bewertung einer Regelabweichung über einen Regelalgorithmus ein Stellsignal an ein Regelstellglied (Dosierpumpe 14) zur Bereitstellung und Zuführung der Regel-Flüssigkeitsmenge abgegeben wird.

5. Betriebsverfahren nach einem der Ansprüche 1 bis 4, **dadurch gekennzeichnet, dass** als Soll-Trockensubstanzgehalt ein Wert kleiner oder gleich 15,5%, vorzugsweise zwischen 10% und 15,5% vorgegeben wird.

6. Betriebsverfahren nach einem der Ansprüche 1 bis 5, **dadurch gekennzeichnet, dass** aus dem der Zudosiereinrichtung (2) unmittelbar nachgeordneten Fermenter (3) und/oder einem diesem Fermenter (3) nachgeordneten Nachfermenter (5) das Substrat für den Separator (8) abgezogen wird.

7. Betriebsverfahren nach Anspruch 6, **dadurch gekennzeichnet, dass** die der zudosierten Stoffmenge entsprechende, dem Fermenter (3) und/oder ggf. dem Nachfermenter (5) entnommene Substratmenge insgesamt dem Separator (8) zugeführt wird und von dem separierten Substrat-Flüssiganteil eine Teilmenge als erforderliche Regel-Flüssigkeitsmenge mittels einer Dosierpumpeinrichtung (14) in den Fermenter (3) zurückgeführt wird, und
dass die Restmenge des Substrat-Flüssigkeitsanteils in einen Nachfermenter (5) oder in ein Endlager (4) überführt und/oder auf Freiflächen ausgebracht wird.

8. Betriebsverfahren nach Anspruch 6, **dadurch gekennzeichnet, dass** die der zudosierten Stoffmenge entsprechende, dem Fermenter (3) und/oder ggf. dem Nachfermenter (5) entnommene Substratmenge nur mit einer solchen Teilmenge dem Separator (8) zugeführt wird und/oder der Separator (8) nur solange angesteuert wird, dass die erforderliche Regel-Flüssigkeitsmenge nach dem Separator (8) zur Verfügung steht und in den Fermenter (3) zurückgeführt wird, und
dass die Restsubstratmenge, die nicht separiert wird, einem Nachfermenter (5) oder einem Endlager (4) zugeführt wird.

9. Betriebsverfahren nach einem der Ansprüche 1 bis 8, **dadurch gekennzeichnet, dass** die Regel-Flüssigkeitsmenge unmittelbar in den Fermenter (3) zurückgeführt und/oder mittelbar zumindest teilweise über eine Vorgrube (15) als Vorratsgefäß und/oder als Anmaischgrube zurückgeführt wird.

10. Betriebsverfahren nach einem der Ansprüche 1 bis 9, **dadurch gekennzeichnet, dass** als Separator (8) ein Schrauben- bzw. Schneckenseparator und/oder ein Zentrifugenseparator und/oder eine Siebbandpresse eingesetzt wird.

11. Betriebsverfahren nach einem der Ansprüche 1 bis 9, **dadurch gekennzeichnet, dass** der Separator (8) eine Schüttung aus Biofeststoffen als Feststofffilter enthält, über die das abgezogene Substrat geleitet wird und unter der der dadurch separierte Substrat-Flüssiganteil (11) für die weitere Verarbeitung aufgefangen wird.

12. Betriebsverfahren nach einem der Ansprüche 1 bis 11, **dadurch gekennzeichnet, dass** der Regelalgorithmus einen Differentialanteil aufweist und/oder mit einer Steuerung für eine schnelle Grobanpassung kombiniert wird.

13. Betriebsverfahren nach einem der Ansprüche 1 bis 12, **dadurch gekennzeichnet, dass** zumindest ein Teil des durch den Separator (8) separierten Substrat-Feststoffanteils (9) über die Zudosiereinrichtung (2) in den Fermenter (3) zurückgeführt wird.

14. Betriebsverfahren nach Anspruch 13, **dadurch gekennzeichnet, dass** der gesamte separierte Substrat-Feststoffanteil (9) wieder vorzugsweise automatisiert über die Zudosiereinrichtung (2) dem Fermenter (3) zugeführt wird und die nicht als Regel-Flüssigkeitsmenge dem Fermenter (3) zurückgeführte Restmenge des am Separator (8) anfallenden Substrat-Flüssigkeitsanteil (11), vorzugsweise automatisiert, einem Endlager (4) zugeführt oder ausgebracht wird.
